# EUROPEAN PATENT APPLICATION

(11) **EP 4 205 823 A1**
(43) Date of publication of application: **05.07.2023**
(21) Application number: 21911276.0
(22) Date of filing: 25.11.2021
(51) Int. Cl.: A63B 24/00, A63B 71/06, G06Q 50/22

(54) **ELECTRONIC DEVICE PROVIDING FITNESS FEEDBACK, CONTROL METHOD FOR SAME, AND SERVER**

(30) Priority: 22.12.2020 KR 20200181277
(71) Applicant: Samsung Electronics Co., Ltd., Suwon-si, Gyeonggi-do 16677 (KR)
(72) Inventor: SON, Taehwan, Suwon-si Gyeonggi-do 16677 (KR); KANG, Seunggu, Suwon-si Gyeonggi-do 16677 (KR); KO, Jihae, Suwon-si Gyeonggi-do 16677 (KR); KIM, Sangmi, Suwon-si Gyeonggi-do 16677 (KR); JUNG, Bosung, Suwon-si Gyeonggi-do 16677 (KR)
(74) Representative: HGF
(86) International application number: PCT/KR2021/017552
(87) International publication number: WO 2022/139209

(57) **Abstract**

Provided is an electronic device. The electronic device comprises at least one sensor, a communication circuit, and at least one processor operatively connected to the communication circuit, wherein the at least one processor may: when exercise information related to an exercise to be performed is received from a server by means of the communication circuit, confirm the motion that each part of the body is to perform on the basis of the exercise information; transmit an activation command on the basis of the confirmed motion to at least one wearable device among a plurality of wearable devices connected by means of the communication circuit; transmit, to the server, at least one of a sensing value sensed by means of the at least one sensor or a sensing value received from the at least one wearable device; and provide feedback received from the server.

## Description

### [Technical Field]

The disclosure relates to an electronic device that provides fitness feedback in real time, a method for controlling the same, and a server.

### [Background Art]

More and more services and additional functions are being provided through electronic devices, e.g., portable electronic devices such as smartphones. For example, home training using an electronic device is taken as a representative method for indoor exercise and ways for achieving maximized home training effects have been sought.

Online fitness systems are mainly used for home training using an electronic device. An online fitness system provider desires to build an online service for effective exercise by providing appropriate feedback to users.

The above information is presented as background information only to assist with an understanding of the disclosure. No determination has been made, and no assertion is made, as to whether any of the above might be applicable as prior art with regard to the disclosure.

### [Disclosure]

### [Technical Problem]

In conventional online fitness systems, the user herself enters information or selects an exercise program, or the system recommends content using entered user information and the user uses the recommended content.

Factors to be considered for maximized exercise effects include selection of a type of exercise considering the correct exercise posture and the user's body characteristics as well as the intensity of exercise.

However, if the user herself enters information, an error may occur depending on subjectivity. Accordingly, if inappropriate content is recommended, the user may lose interest in exercise or give up in the middle.

Further, as inappropriate content is recommended and non-personalized general-purpose feedback is provided to the user, the risk of injury to the user may increase.

Aspects of the disclosure are to address at least the above-mentioned problems and/or disadvantages and to provide at least the advantages described below. Accordingly, an aspect of the disclosure is to provide an electronic device that provides fitness feedback in real time, a method for controlling the same, and a server.

Additional aspects will be set forth in part in the description which follows and, in part, will be apparent from the description, or may be learned by practice of the presented embodiments.

### [Technical Solution]

In accordance with an aspect of the disclosure, an electronic device is provided. The electronic device includes at least one sensor, a communication circuitry, and at least one processor operatively connected with the at least one sensor and the communication circuitry, wherein the at least one processor may be configured to, if exercise information related to an exercise to be performed is received from a server through the communication circuitry, identify a movement for each part of a body to be performed based on the exercise information, transmit an activation command to at least one of a plurality of connected wearable devices through the communication circuitry based on the identified movement, transmit, to the server, at least one of a sensing value detected through the at least one sensor or a sensing value received from the at least one wearable device, and provide feedback received from the server.

In accordance with another aspect of the disclosure, a method for controlling an electronic device is provided. The method includes, if exercise information related to an exercise to be performed is received from a server, identifying a movement for each part of a body to be performed based on the exercise information, transmitting an activation command to at least one of a plurality of connected wearable devices based on the identified movement, transmitting, to the server, at least one of a sensing value detected through at least one sensor or a sensing value received from the at least one wearable device, and providing feedback received from the server.

In accordance with another aspect of the disclosure, a server is provided. The server includes a memory, a communication circuitry, and at least one processor operatively connected with the memory and the communication circuitry, wherein the at least one processor may be configured to identify an exercise to be performed based on a first user's account, transmit exercise information including information related to a movement for each body part of the exercise to be performed through the communication circuitry to the first user's electronic device, receive at least one of a sensing value or the first user's exercise information from the first user's electronic device, transmit at least one of the received sensing value or the first user's exercise image to a second user's electronic device and, upon receiving feedback information from the second user's electronic device, transmit the feedback information to the first user's electronic device.

### [Advantageous Effects]

According to various embodiments of the disclosure, in the electronic device, as the multimedia information and the exercise-related information are transmitted through separate channels, the server that processes the multimedia information and the server that processes the exercise-related information may be separated from each other, thus, it is possible to enhance security for exercise-related information, which is related to personal privacy, such as biometric information, and to provide an enhanced real-time service as the speed of transmission/reception of sensing information and feedback increases.

Further, according to various embodiments of the disclosure, the electronic device may obtain the sensing value for each body part, thus allowing for more accurate activity recognition.

Further, according to various embodiments of the disclosure, the electronic device may adjust the exercise intensity through real-time feedback, thus maximizing exercise efficiency and preventing injury.

Other aspects, advantages, and salient features of the disclosure will become apparent to those skilled in the art from the following detailed description, which, taken in conjunction with the annexed drawings, discloses various embodiments of the disclosure.

### [Description of the Drawings]

The above and other aspects, features, and advantages of certain embodiments of the disclosure will be more apparent from the following description taken in conjunction with the accompanying drawings, in which:
FIG. 1 is a view illustrating an electronic device in a network environment according to an embodiment of the disclosure;
FIG. 2 is a view illustrating communication connections of electronic devices according to an embodiment of the disclosure;
FIG. 3 is a view illustrating communication connections of electronic devices according to an embodiment of the disclosure;
FIG. 4A is a view illustrating operations of a trainer's electronic device according to an embodiment of the disclosure;
FIG. 4B is a view illustrating a configuration of an exercise program included in a fitness service according to an embodiment of the disclosure;
FIG. 5A is a view illustrating operations of a trainee's electronic device according to an embodiment of the disclosure;
FIG. 5B is a view illustrating exercise content provided to a trainee according to an embodiment of the disclosure;
FIG. 6 is a view illustrating operations of a trainee's electronic device according to an embodiment of the disclosure;
FIG. 7 is a view illustrating a movement for each body part in exercise to be performed according to an embodiment of the disclosure;
FIG. 8A is a view illustrating a reference sensing value for exercise to be performed according to an embodiment of the disclosure;
FIG. 8B is a view illustrating a sensing value obtained according to a trainee's movement according to an embodiment of the disclosure;
FIG. 9A is a view illustrating a feedback providing operation according to an embodiment of the disclosure;
FIG. 9B is a view illustrating a feedback providing operation according to an embodiment of the disclosure;
FIG. 9C is a view illustrating a feedback providing operation according to an embodiment of the disclosure; and
FIG. 10 is a view illustrating a real-time feedback providing operation according to an embodiment of the disclosure.

Throughout the drawings, it should be noted that like reference numbers are used to depict the same or similar elements, features, and structures.

### [Mode for Invention]

The following description with reference to the accompanying drawings is provided to assist in a comprehensive understanding of various embodiments of the disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

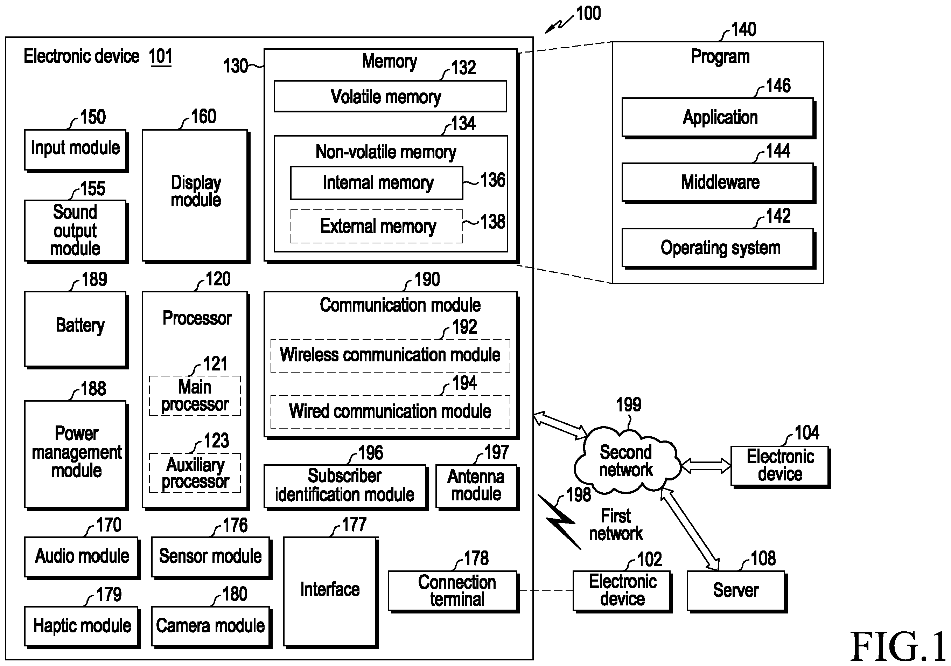

FIG. 1 is a block diagram illustrating an electronic device in a network environment according to an embodiment of the disclosure. Referring to FIG. 1, an electronic device 101 in a network environment 100 may communicate with at least one of an electronic device 102 via a first network 198 (e.g., a short-range wireless communication network), or an electronic device 104 or a server 106 via a second network 199 (e.g., a long-range wireless communication network). According to an embodiment, the electronic device 101 may communicate with the electronic device 104 via the server 106. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one (e.g., the connecting terminal 178) of the components may be omitted from the electronic device 101, or one or more other components may be added in the electronic device 101. According to an embodiment, some (e.g., the sensor module 176, the camera module 180, or the antenna module 197) of the components may be integrated into a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be configured to use lower power than the main processor 121 or to be specified for a designated function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. The artificial intelligence model may be generated via machine learning. Such learning may be performed, e.g., by the electronic device 101 where the artificial intelligence is performed or via a separate server (e.g., the server 106). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, keys (e.g., buttons), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor configured to detect a touch, or a pressure sensor configured to measure the intensity of a force generated by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or motion) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 106) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device 104 via a first network 198 (e.g., a short-range communication network, such as Bluetooth^{™}, wireless-fidelity (Wi-Fi) direct, or infrared data association (IrDA)) or a second network 199 (e.g., a long-range communication network, such as a legacy cellular network, a 5G network, a next-generation communication network, the Internet, or a computer network (e.g., local area network (LAN) or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device). According to an embodiment, the antenna module 197 may include one antenna including a radiator formed of a conductor or conductive pattern formed on a substrate (e.g., a printed circuit board (PCB)). According to an embodiment, the antenna module 197 may include a plurality of antennas (e.g., an antenna array). In this case, at least one antenna appropriate for a communication scheme used in a communication network, such as the first network 198 or the second network 199, may be selected from the plurality of antennas by, e.g., the communication module 190. The signal or the power may then be transmitted or received between the communication module 190 and the external electronic device via the selected at least one antenna. According to an embodiment, other parts (e.g., radio frequency integrated circuit (RFIC)) than the radiator may be further formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 106 coupled with the second network 199. The external electronic devices 102 or 104 each may be a device of the same or a different type from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra-low-latency services using, e.g., distributed computing or mobile edge computing. In another embodiment, the external electronic device 104 may include an internet-of-things (IoT) device. The server 106 may be an intelligent server using machine learning and/or a neural network. According to an embodiment, the external electronic device 104 or the server 106 may be included in the second network 199. The electronic device 101 may be applied to intelligent services (e.g., smart home, smart city, smart car, or health-care) based on 5G communication technology or IoT-related technology.

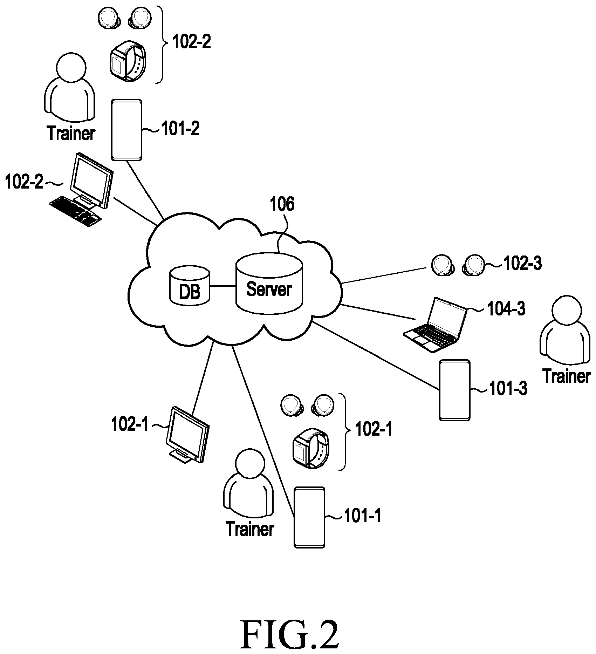

FIG. 2 is a view illustrating communication connections of electronic devices according to an embodiment of the disclosure.

Referring to FIG. 2, a server 106 (e.g., the server 106 of FIG. 1) of a fitness service system may communicate with a plurality of electronic devices 101-1, 101-2, and 101-3 (e.g., the electronic device 101 of FIG. 1). For example, the plurality of electronic devices 101-1, 101-2, and 101-3 may include a first user's (or a trainee's) electronic devices 101-1 and 101-3 and a second user's (or a trainer's) electronic device 101-2. According to various embodiments, the plurality of electronic devices 101-1, 101-2, and 101-3 may be user terminal devices (e.g., smartphones) on which applications related to fitness services may be installed and executed.

According to various embodiments, the server 106 may include a memory storing a database DB, a communication module for communicating with external devices, and at least one processor operatively connected with the memory and the communication module.

According to various embodiments, the electronic device 101-1, 101-2, or 101-3 may include a plurality of sensors and may be mounted on the user's body to obtain a sensed value related to the user's movement.

According to various embodiments, the first user and the second user may include a plurality of external devices together with the electronic device 101-1, 101-2, or 101-3. According to various embodiments, the plurality of external devices may include at least one wearable device 102-1, 102-2, or 102-3 (e.g., the electronic device 102 of FIG. 1) and an external display device 104-1, 104-2, or 104-3 (e.g., the electronic device 104 of FIG. 1). For example, the at least one wearable device 102-1, 102-2, or 102-3 may be a device mounted on the body of the first user or the second user. For example, at least one of an earphone, a headset, a head mounted display (HMD) device, smart glasses, or a hat may be mounted on the head of the first user or the second user. At least one of a smart watch, a smart band, a ring, a patch or a belt may be mounted on the torso of the first user or the second user. At least one of a shoe, a knee band, and a smartphone may be mounted on the first user's or second user's leg (e.g., a smartphone may be fixed with a band or positioned in a pocket). However, the devices are not limited thereto, as long as they may be mounted on the user's body. According to various embodiments, at least one wearable device 102-1, 102-2, or 102-3 may include a plurality of sensors and may be mounted on the user's body to obtain sensing values related to the user's movement.

According to various embodiments, sensors included in the electronic device 101-1, 101-2, or 101-3 or at least one wearable device 102-1, 102-2, or 102-3 may include at least one of motion sensors, barometer sensors, or biometric sensors.

For example, the motion sensors may include an accelerometer, a gyroscope, and/or a magnetic sensor. The barometer sensor may detect a change in altitude using a change in the difference in air pressure according to the altitude. The biometric sensor may include a photoplethysmogram (PPG) sensor and/or an electrode sensor and may obtain the user's biometric information, such as heart rate, electrocardiogram, sweat, body water, body composition, blood pressure, blood sugar, oxygen saturation, and body temperature.

According to various embodiments, the external display device 104-1, 104-2, or 104-3 (e.g., the electronic device 104 of FIG. 1) may include a smart TV, monitor, laptop PC, or tablet PC capable of communicating with the server 106 or the electronic device 101-1, 101- 2 or 101-3.

According to various embodiments, at least one wearable device 102-1, 102-2, or 102-3 may be connected with the electronic device 101-1, 101-2, or 101-3 or may be connected with the server 106. For example, if at least one wearable device 102-1, 102-2, or 102-3 is connected with the electronic device 101-1, 101-2, or 101-3, the electronic device 101-1, 101-2 or 101-3 may gather the sensing values obtained from at least one wearable device 102-1, 102-2, or 102-3, transmit the gathered sensing values to the server 106, and transmit data, such as a control commands, to the at least one wearable device 102-1, 102-2 or 102-3 based on the information received from the server 106. In another embodiment, when at least one wearable device 102-1, 102-2, or 102-3 is connected with the server 106, at least one wearable device 102-1, 102-2, or 102-3 may transmit the obtained sensing value to the server 106, and the server 106 may transmit data, such as a control command, to the at least one wearable device 102-1, 102-2 or 102-3.

According to various embodiments, the external display device 104-1, 104-2, or 104-3 may be connected with the electronic device 101-1, 101-2, or 101-3, or may be connected with the server 106. According to various embodiments, when a display (e.g., the display module 160 of FIG. 1) included in the electronic device 101-1, 101-2, or 101-3 is used to provide a fitness service, the external display device 104-1, 104-2, or 104-3 may be omitted.

According to various embodiments, at least one wearable device 102-1, 102-2, or 102-3 may be interconnected. For example, an earphone and a smart watch, which are at least one wearable device 102-1 of the first user, may be connected with each other to perform communication.

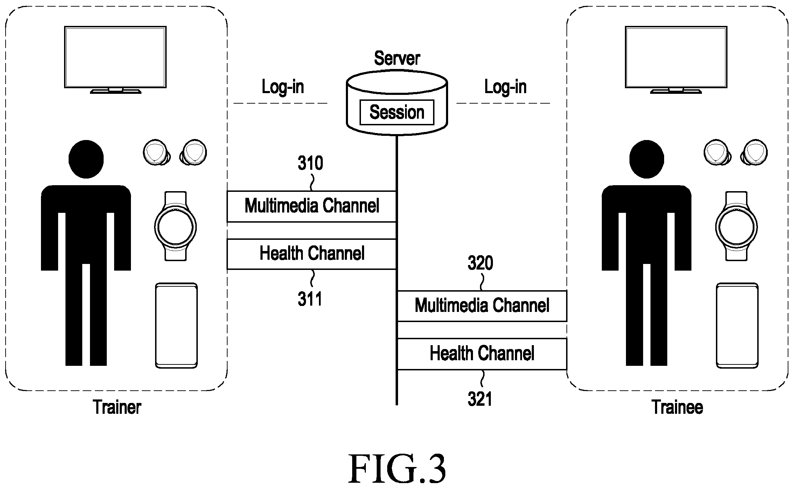

FIG. 3 is a view illustrating communication connections of electronic devices according to an embodiment of the disclosure.

Referring to FIG. 3, a fitness service system may include a trainer's device group (e.g., the electronic device 101-2, the wearable device 102-2, and/or the external display device 104-2 of FIG. 2) and a trainee's device group (e.g., the electronic device 101-1, the wearable device 102-1, and/or the external display device 104-1 of FIG. 2) connected via a server (e.g., the server 106 of FIG. 1 or the server 106 of FIG. 2). For example, each of the trainer's device group and the trainee's device group may include an electronic device (e.g., a smartphone) (e.g., the electronic device 101 of FIG. 1 or the electronic device 101-1 or 101-2 of FIG. 2) capable of executing a fitness service application, at least one wearable device (e.g., the electronic device 102 of FIG. 1 or the wearable device 102-1 or 102-2 of FIG. 2) capable of detecting the user's movement, and an external display device (e.g., the electronic device 104 of FIG. 1 or the external display device 104-1 of 104-2 of FIG. 2) capable of providing an image. According to various embodiments, the electronic device (e.g., the electronic device 101 of FIG. 1 or the electronic device 101-1 or 101-2 of FIG. 2) may also detect the user's movement. If the fitness image may be provided through the display (i.e., display module 160 of FIG. 1) included in the electronic device (e.g., the electronic device 101 of FIG. 1 or the electronic device 101-1 or 101-2 of FIG. 2), the external display device may be omitted.

According to various embodiments, the plurality of devices included in the trainer's device group and the plurality of devices included in the trainee's device group may be logged into the fitness service provided by the server through the trainer's account or the trainee's account, and the information obtained from each of the plurality of devices included in the device group may be transmitted to the server. According to various embodiments, the wearable device or the external display device included in the device group is connected with a server directly or through an electronic device (e.g., the electronic device 101 of FIG. 1 or the electronic device 101-1 or 101-2 of FIG. 2).

According to various embodiments, when the trainer's electronic device and the trainee's electronic device are connected to the session created by the server for real-time provision of a fitness service, multimedia information, such as images, audio, and text, as well as the exercise information and sensing values may also be transmitted/received between the server and the electronic device through the session. For example, session refers to an exercise course of one session included in an exercise program performed for a plurality of sessions over several days, and one session may be composed of a combination of a plurality of exercise movements.

According to various embodiments, the trainer may create a virtual session in the server or log in with an access right, and trainee may log in with the permission to access the already created session.

According to various embodiments, the fitness service system may transmit/receive information through multiple channels to provide a real-time service. For example, the fitness service system may transmit/receive information using a multimedia channel 310 and a health channel 311 between the trainer's electronic device and the server and may transmit/receive information using a multimedia channel 320 and a health channel 321 between the server and the trainee's electronic device.

According to various embodiments, the multimedia channels 310 and 320 may be channels for transmitting and receiving images, voices, and texts. For example, the multimedia channels 310 and 320 may transmit and receive information by way of, e.g., video calls, voice calls, and chatting.

According to various embodiments, an input received through a camera, microphone, or input interface (e.g., a touch panel or button) included in a device group of a user (e.g., the trainer or trainee) may be transmitted through the multimedia channel 310 or 320 to the server or may be transferred through the server to the device group of the other user (e.g., the trainee or trainer). In another embodiment, multimedia information transmitted from the other user's device group to the server may be displayed through an external display device included in the user's device group through the server.

According to various embodiments, the multimedia channels 310 and 320 may be configured as a single channel or separate channels for video and audio, and the server may control to receive multimedia information from what device or to transmit multimedia information to what device based on the performance of the devices.

According to various embodiments, the health channels 311 and 321 may be channels for transmitting and receiving, in real time, information related to a program generated in a session, a command designated by the trainer or trainee, and the sensing values obtained from the devices included in the device group. For example, the designated command may be a command related to the program created in the session and may include the progress time, the number of times, image playback/pause, or feedback information for the exercise generated by the trainer.

According to various embodiments, the sensing value resultant from sensing the movement of the trainer or trainee may be transmitted/received, as raw data, to/from the server through the health channel 311 or 321 or may be transmitted/received, as information image-processed based on the raw data, to/from the server.

As described above, as the multimedia information and the exercise-related information are transmitted through separate channels, the server that processes the multimedia information and the server that processes the exercise-related information may be separated from each other. Thus, it is possible to enhance security for exercise-related information, which is related to personal privacy, such as biometric information, and to provide an enhanced real-time service as the speed of transmission/reception of sensing information and feedback increases.

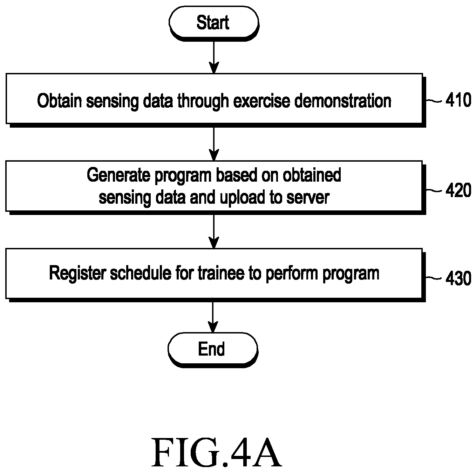

FIG. 4A is a view illustrating operations of a trainer's electronic device according to an embodiment of the disclosure.

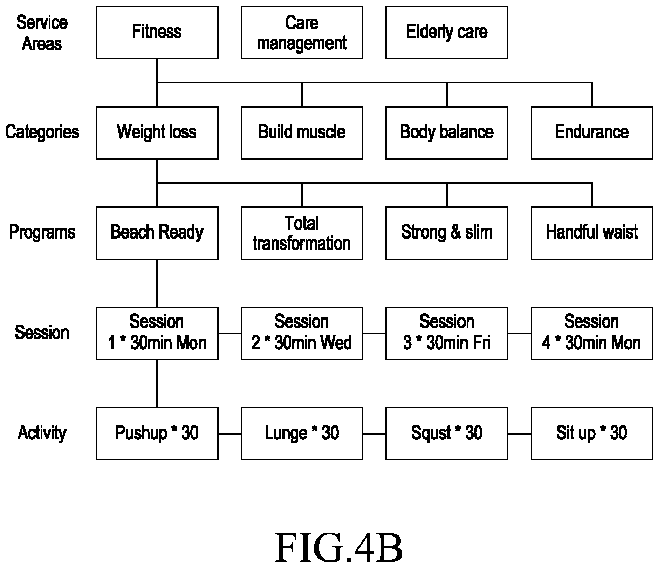

FIG. 4B is a view illustrating a configuration of an exercise program included in a fitness service according to an embodiment of the disclosure.

Referring to FIG. 4A, in operation 410, the trainer's electronic device (e.g., the electronic device 101 of FIG. 1, the processor 120 of FIG. 1, or the electronic device 101-2 of FIG. 2) may obtain sensing data through the trainer's exercise demonstration. For example, the trainer may perform an exercise demonstration after wearing an electronic device or a wearable device (e.g., the wearable device 102 - 2 of FIG. 2) on her body part, and the trainer's electronic device (i.e., electronic device 101) may obtain sensing data obtained from a sensor included in the electronic device 101 or a sensor included in a wearable device connected with the electronic device 101.

According to various embodiments, the trainer's electronic device (i.e., electronic device 101) may obtain an image of the trainer's exercise demonstration through a camera included in the electronic device 101 or an external camera device connected with the electronic device 101.

According to various embodiments, in operation 420, the trainer's electronic device (i.e., electronic device 101) may generate a program based on the obtained sensing data and upload the generated program to a server (e.g., the server 106 of FIG. 1 or the server 106 of FIG. 2).

According to various embodiments, the trainer's electronic device (i.e., electronic device 101) may configure the following program based on the trainer's manipulation and upload it to the server. For example, the exercise program may include a configuration of the exercise program as illustrated in FIG. 4B.

Referring to FIG. 4B, a fitness service may include a plurality of categories for each of a plurality of service areas and a plurality of programs for each category, and each program may consist of a plurality of sessions, and each session may include a combination of a plurality of activities. For example, the program may include information regarding the exercise time for each session and the number of movements included in the session.

FIG. 4B merely illustrates an example of an exercise program, but embodiments of the disclosure are not limited thereto.

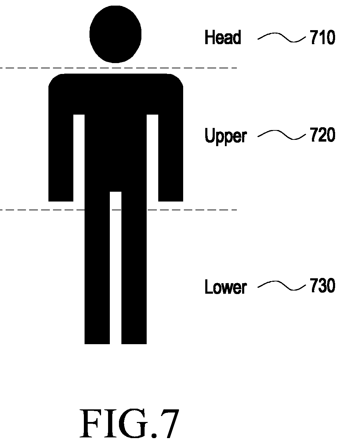

According to various embodiments, each session may include a combination of a plurality of activities, and each activity may be composed of a movement for each body part. According to various embodiments, the movement of each body part may be classified into a type of movement that may be sensed through at least one of a sensor of the trainer's electronic device worn on the body or a sensor of a wearable device. The movement of each body part constituting the activity is described below with reference to FIG. 7.

According to various embodiments, a session included in a program may include a form as shown in Table 1 below. The form of the session in Table 1 below is merely an example and is not limited thereto.

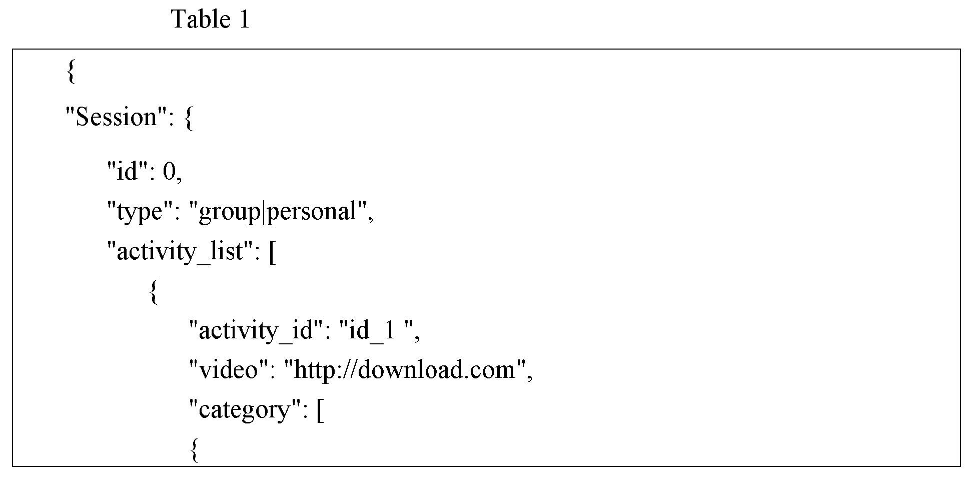

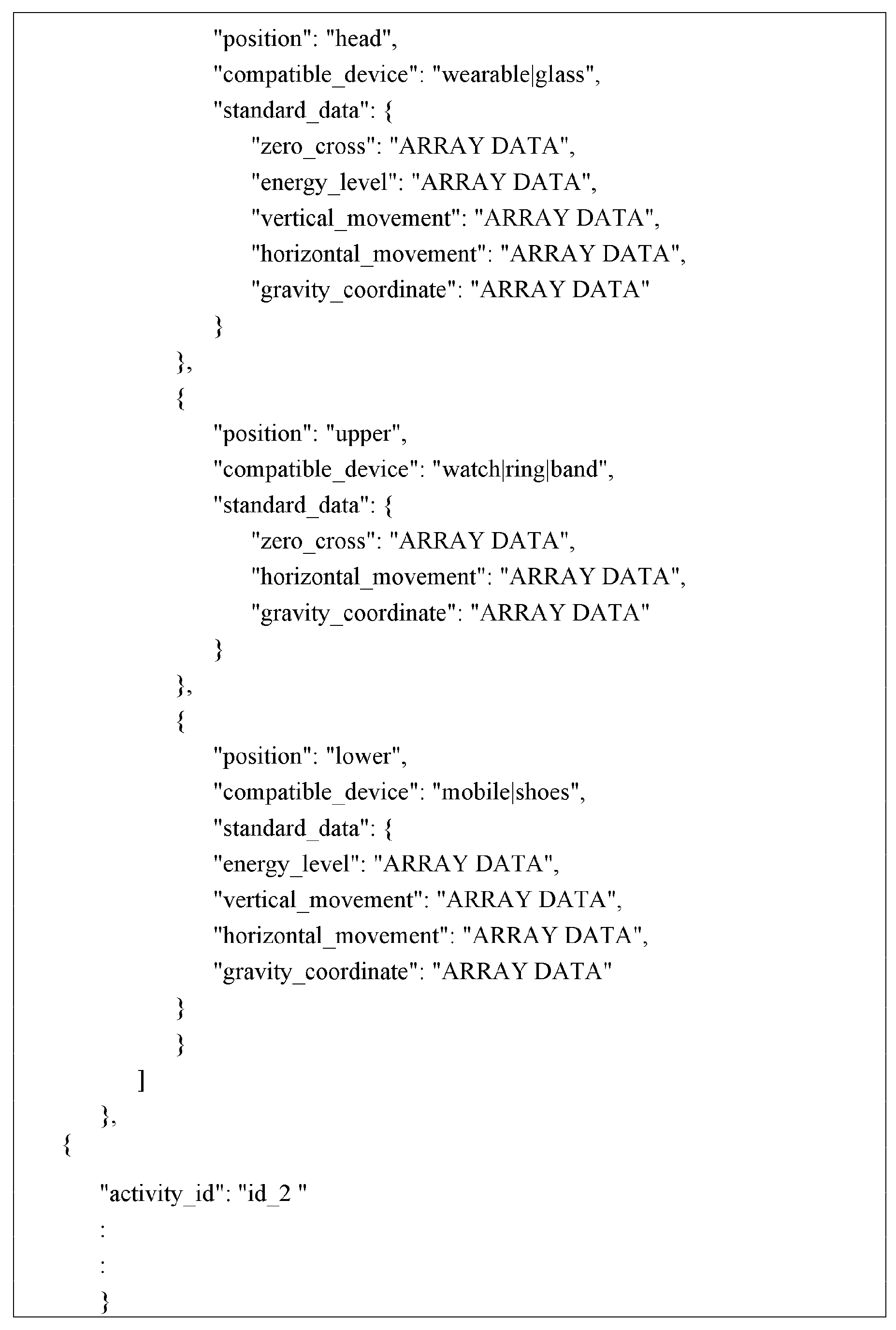

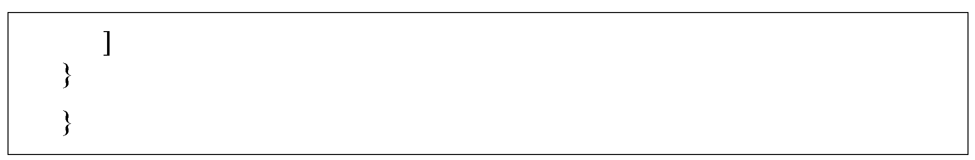

According to various embodiments, a session is a group of activities that are the minimum exercise units and may mean a combination of activities to be continuously performed. According to various embodiments, 'id' may mean a unique ID added when a trainer creates a program, and 'type' is a value that sets whether to expose the trainee when the corresponding activity is performed, which may mean whether to disclose a session created in real time. According to various embodiments, 'activity list' may mean a minimum unit of exercise to be performed in a corresponding session, and 'activity id' may mean a unique ID of the corresponding activity. According to various embodiments, 'video' may mean guide content information for a reference activity for a corresponding activity, and 'category' may mean information regarding the position of a device required to monitor the sensing value when a corresponding activity is performed. For example, when the body parts are divided into Head, Upper, and Lower, electronic devices that may be positioned on the Head may include an earphone, glasses, a headset, a neckband, an HMD, and a hat, electronic devices that may be positioned on the Upper may include a watch, a band, a smartphone, a chest patch, smart wear, and a ring, and electronic devices that may be positioned on the Lower may include a smartphone, shoes, and smart wear.

According to various embodiments, 'compatible device' means a device that may measure the sensor value in the position designated in the 'category', and 'standard data' is a reference value for the data to be measured by the sensor for each 'category' when each activity is performed and may mean sensing information for each movement type. According to various embodiments, a weight (considering a main activity or timing) may be given according to a time stamp for each reference activity of 'video', and the fitness service system (e.g., the electronic device 101-1 or 101-2 or the server 106 of FIG. 2) may evaluate the 'standard' and the accuracy of the user's movement sensing value and the exercise effect.

According to various embodiments, the trainer's electronic device (i.e., electronic device 101) may upload the generated program to the server, and limit the scope of distribution by setting the right to look up the whole or part of the program.

According to various embodiments, the trainer's electronic device (i.e., electronic device 101) may register a schedule for the trainee to perform a program in operation 430. For example, if the trainer's electronic device (i.e., electronic device 101) registers the schedule for the trainee to perform a program in the server, the server may transmit a notification to the trainee's electronic device (e.g., the electronic device 101 of FIG. 1 or the electronic device 101-1 or 101-3 of FIG. 2) in the registered schedule.

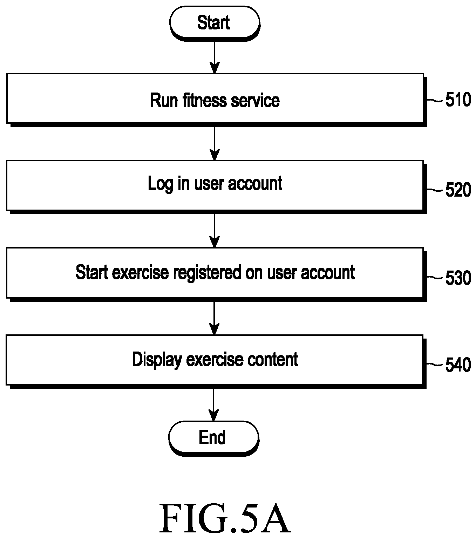

FIG. 5A is a view illustrating operations of a trainee's electronic device according to an embodiment of the disclosure.

Referring to FIG. 5A, in operation 510, a trainee's electronic device (e.g., the electronic device 101 of FIG. 1, the processor 120 of FIG. 1, or the electronic device 101-1 or 101-3 of FIG. 2) may run a fitness service. For example, the trainee's electronic device (i.e., electronic device 101) may execute a fitness service application by the trainee's manipulation. According to various embodiments, the fitness service may be performed by the trainee's manipulation after a notification is provided to the trainee according to the schedule registered by the trainer or may be performed by the trainee's arbitrary manipulation regardless of the notification.

According to various embodiments, in operation 520, the trainee's electronic device (i.e., electronic device 101) may perform user account login. For example, the trainee's electronic device (i.e., electronic device 101) may access the server by logging in using the trainee's user account registered in the server.

According to various embodiments, in operation 530, the trainee's electronic device (i.e., electronic device 101) may start an exercise registered in the user account. For example, the exercise registered in the trainee's user account may be included in the exercise program selected by the trainee or may be set by the trainer.

According to various embodiments, in operation 540, the trainee's electronic device (i.e., electronic device 101) may display exercise content. For example, the trainee's electronic device (i.e., electronic device 101) may display exercise content for guiding a reference activity of the started exercise. According to various embodiments, the trainee's electronic device (i.e., electronic device 101) may receive and display the exercise content from a server (e.g., the server 106 of FIG. 1 or the server 106 of FIG. 2), and the exercise content may be an exercise-related image uploaded to the server by the trainer in real time or an image already stored in the server.

According to various embodiments, the exercise content may be an image obtained by the trainer through a camera during exercise demonstration or an image image-processed based on the sensing value from the wearable device worn on the trainer's body part.

According to various embodiments, the trainee's electronic device (i.e., electronic device 101) may display the exercise content through a display (e.g., the display module 160 of FIG. 1) included in the electronic device 101 or an external display device (e.g., the electronic device 104 of FIG. 1 or the external display device 104-2 or 104-3 of FIG. 2) connected through the communication module (e.g., the communication module 190 of FIG. 1) of the electronic device 101.

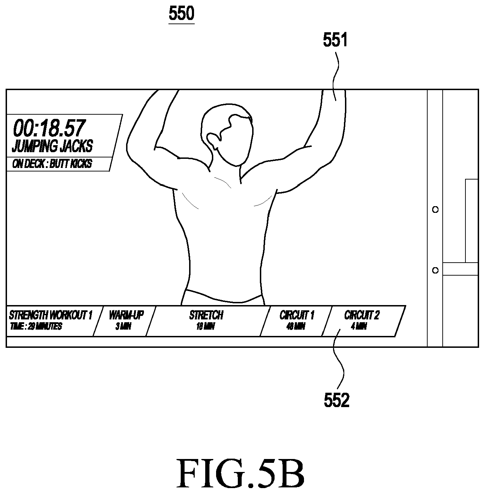

FIG. 5B is a view illustrating exercise content provided to a trainee according to an embodiment of the disclosure.

Referring to FIG. 5B, the trainee's electronic device (e.g., the electronic device 101 of FIG. 1 or the electronic device 101-1 or 101-3 of FIG. 2) may display exercise content 550 as the fitness service application starts an exercise. For example, the exercise content 550 may include an exercise demonstration image 551 of a trainer and information 552 related to the exercise to be performed. The information 552 related to the exercise to be performed may include a plurality of exercise activities included in the session, the order of the exercise activities, the number of exercises and the exercise time related to the exercise intensity for each exercise activity.

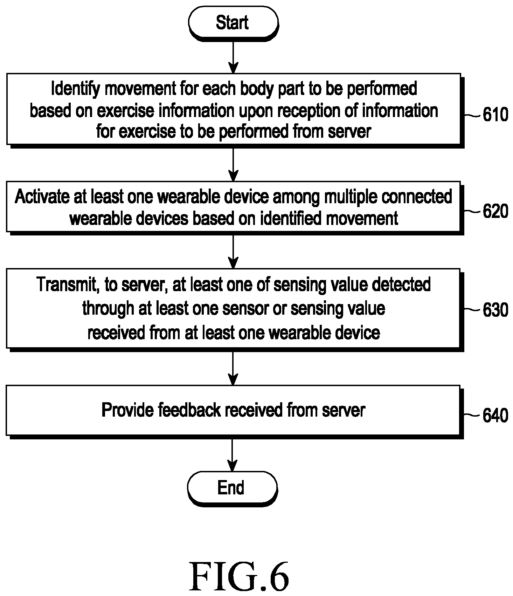

FIG. 6 is a view illustrating operations of a trainee's electronic device according to an embodiment of the disclosure.

Referring to FIG. 6, in operation 610, a trainee's electronic device (e.g., the electronic device 101 of FIG. 1, the processor 120 of FIG. 1, or the electronic device 101-1 or 101-3 of FIG. 2), upon receiving information about an exercise to be performed from a server (e.g., the server 106 of FIG. 1 or the server 106 of FIG. 2), may identify the movement of each body part to be performed based on the exercise information.

For example, the trainee's electronic device (i.e., electronic device 101) may divide the trainee's body into a plurality of parts and may identify the type of at least one movement included in each of the plurality of parts based on the exercise information received from the server. The operation of dividing the body into parts and the type of the movement for each part of the exercise to be performed are described below with reference to FIG. 7.

According to various embodiments, the exercise information received from the server may include types of a plurality of activities included in the session to be performed by the trainee, the order of the plurality of activities, exercise intensity information related to the exercise time and number of times of each activity, and/or an image for guiding the reference activity.

According to various embodiments, the trainee's electronic device (i.e., electronic device 101) may identify the movement for each body part for the activity to be performed by the trainee among a plurality of activities included in the received exercise information.

According to various embodiments, in operation 620, the trainee's electronic device (i.e., electronic device 101) may activate at least one wearable device among a plurality of connected wearable devices (e.g., the wearable device 102-1 or 102-3 of FIG. 2) based on the identified movement. For example, the trainee's electronic device (i.e., electronic device 101) may identify the type of movement for each of the plurality of parts and may transmit an activation command to at least one wearable device capable of detecting the identified type of the movement for each of the plurality of parts among the plurality of wearable devices connected with the trainee's electronic device (i.e., electronic device 101).

In another embodiment, the trainee's electronic device (i.e., electronic device 101) may transmit a command for activating at least one sensor of at least one wearable device capable of detecting the identified type of movement for each of the plurality of parts to the at least one wearable device.

According to various embodiments, if at least one sensor (e.g., the sensor module 176 of FIG. 1) included in the trainee's electronic device (i.e., electronic device 101) may detect the identified type of movement for each of the plurality of parts, the trainee's electronic device (i.e., electronic device 101) may activate at least one sensor included therein.

According to various embodiments, in operation 630, the trainee's electronic device (i.e., electronic device 101) may transmit, to the server, at least one of the sensing value detected through at least one sensor (e.g., the sensor module 176 of FIG. 1) or the sensing value received from at least one wearable device. For example, the trainee's electronic device (i.e., electronic device 101) may transmit at least one of the sensing value detected through at least one sensor or the sensing value received from at least one wearable device, as raw data, to the server, through a communication module (e.g., the communication module 190 of FIG. 1).

As another embodiment, the trainee's electronic device (i.e., electronic device 101) may transmit information image-processed based on the raw data of the sensing value to the server. For example, the trainee's electronic device (i.e., electronic device 101) may generate a movement image of the trainee based on the raw data of the sensing value and may transmit the generated trainee's movement image to the server.

As another embodiment, the trainee's electronic device (i.e., electronic device 101) may identify a reference sensing value corresponding to the type of movement for each body part included in the exercise information received from the server and may transmit, to the server, a result of comparison between at least one of the sensing value detected through at least one sensor or the sensing value received from at least one wearable device and the reference sensing value.

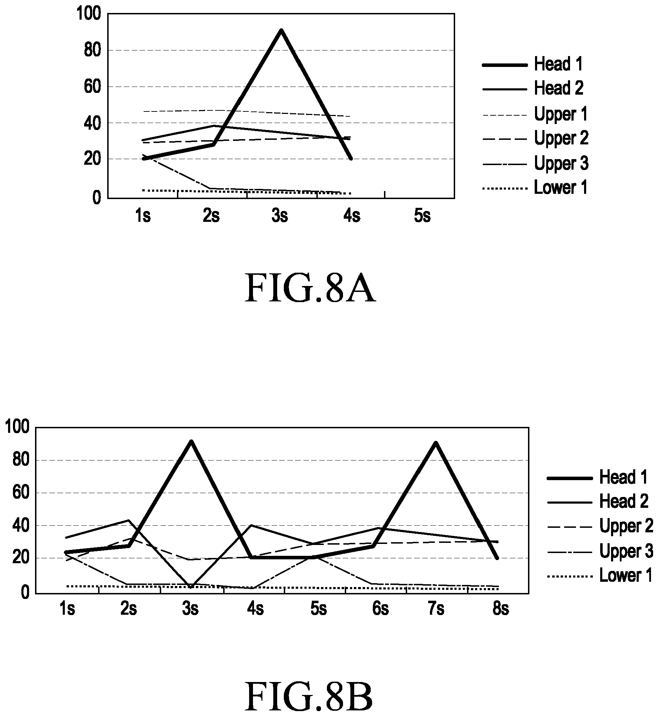

For example, the trainee's electronic device (i.e., electronic device 101) may compare the reference sensing value for the exercise to be performed as illustrated in FIG. 8A with the sensing value obtained according to the movement of the trainee as illustrated in FIG. 8B, thereby obtaining the result of comparison.

FIG. 8A is a view illustrating a reference sensing value for exercise to be performed according to an embodiment of the disclosure.

FIG. 8B is a view illustrating a sensing value obtained according to a trainee's movement according to an embodiment of the disclosure.

Referring to FIGS. 8A and 8B, sensing values that may be obtained in relation to the activity performed by the trainee may include the sensing values from two sensors related to the head, three sensing values related to the upper body, and one sensing value related to the lower body. The sensing values may be detected by different sensors, and the sensors may be included in the same wearable device or different wearable devices.

According to various embodiments, the reference sensing values as illustrated in FIG. 8A may be sensing values obtained from a plurality of sensors included in the trainer's exercise device (e.g., the electronic device 101-2 of FIG. 2) or wearable device (e.g., the electronic device 101-2 of FIG. 2) by the exercise demonstration conducted by the trainer wearing the wearable device or may be values stored in the server.

According to various embodiments, the trainee's electronic device (i.e., electronic device 101) may compare the reference sensing value with the sensing value according to the trainee's movement and, if a preset similarity is met, identify that the activity has been performed and may identify the number of activities performed through the number of repetitions of the pattern included in the sensing value according to the trainee's movement.

Although FIG. 6 illustrates that the comparison between the reference sensing value and the sensing value according to the trainee's movement is performed by the trainee's electronic device (i.e., electronic device 101), according to various embodiments, the server may receive the sensing value according to the trainee's movement and perform the comparison.

According to various embodiments, the trainee's electronic device (i.e., electronic device 101) may transmit, through a first channel (e.g., the health channel 321 of FIG. 3) to the server, at least one of the sensing value detected through at least one sensor or the sensing value received from at least one wearable device.

According to various embodiments, the trainee's electronic device (i.e., electronic device 101) may transmit, through a second channel (e.g., the multimedia channel 320 of FIG. 3) different from the first channel to the server, data for the trainee's exercise image received from the camera (e.g., the camera module 180 of FIG. 1) included in the trainee's electronic device (i.e., electronic device 101) or an external camera, data for the trainee's exercise image generated based on the raw data, the trainee's audio data received from the microphone (e.g., the input module 150 of FIG. 1) included in the trainee's electronic device (i.e., electronic device 101) or an external microphone, and text data entered by the trainee.

According to various embodiments, in operation 640, the trainee's electronic device (i.e., electronic device 101) may provide the feedback received from the server. For example, the feedback may include at least one of voice feedback or visual feedback related to at least one of posture correction or exercise intensity of the activity being performed by the trainee.

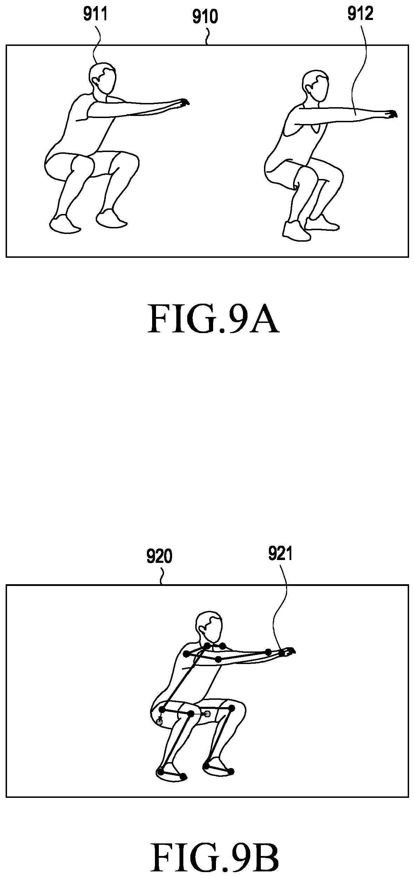

For example, the visual feedback may be an image for posture correction of the activity being performed, and various embodiments of the visual feedback are described below with reference to FIGS. 9A to 9C. As another embodiment, if the exercise intensity, such as the number of activities or the exercise time, is adjusted, the adjusted number or time may be included in the feedback image and provided.

According to various embodiments, upon receiving visual feedback from the server, the trainee's electronic device (i.e., electronic device 101) may display the visual feedback through a display included in the trainee's electronic device (i.e., electronic device 101) or may transmit the visual feedback to a connected external display device to allow the external display device to display the visual feedback.

According to various embodiments, the voice feedback may be the trainer's voice related to posture correction or exercise intensity. The trainee's electronic device (i.e., electronic device 101) may output the voice feedback through the speaker (e.g., the sound output module 155 of FIG. 1) included in the trainee's electronic device (i.e., electronic device 101) or transmit the voice feedback to an external speaker device to allow the external speaker device to output the voice feedback.

According to various embodiments, the feedback may be input from an external device connected with the server or stored in the server. For example, the feedback may be input in real time from the trainer's electronic device (e.g., the electronic device 101-2 of FIG. 2) connected with the server and transmitted to the server or may be previously stored in the server.

According to various embodiments, the trainee's electronic device (i.e., electronic device 101) may transmit a control command for providing vibration feedback to at least some of at least one wearable device activated based on the feedback received from the server. For example, the trainee's electronic device (i.e., electronic device 101) may identify a wearable device worn near a body part requiring posture correction among at least one activated wearable device based on the feedback received from the server and may transmit a control command to generate vibration to the wearable device worn near the body part requiring posture correction. As such, it is possible to provide more intuitive feedback to the trainee by providing vibration feedback to the body part requiring posture correction.

FIG. 7 is a view illustrating movement for each body part in exercise to be performed according to an embodiment of the disclosure.

Referring to FIG. 7, according to various embodiments, an electronic device (e.g., the electronic device 101 of FIG. 1, the processor 120 of FIG. 1, or the electronic device 101-1 or 101-3 of FIG. 2, or the electronic device 101-2 of FIG. 2) may divide the body of the user (e.g., a trainer or a trainee) into a plurality of parts. For example, the electronic device 101 may divide the user's body into a head 710, an upper body 720, or a lower body 730. This is merely an embodiment, and the user's body may be divided into two or four or more.

According to various embodiments, the electronic device 101 may divide types of movements detectable through at least one sensor (e.g., a motion sensor, a barometer sensor, a gyro sensor, a geomagnetic sensor, or an acceleration sensor) into zero-cross, energy level, vertical movement, horizontal movement, and gravity coordinate.

For example, zero cross is an exercise that reciprocates while accompanied by rotation and may correspond to a fly, jump rope, or curl activity.

For example, energy level may determine the amount of activity using the magnitude of the acceleration generated by the movement and may also be defined as the amount of impact and may correspond to a jumping jack, a plank, or a pitch activity.

For example, vertical movement is a vertical movement exercise, which may correspond to a squat, a lat pull down, or a bench press.

For example, horizontal movement is a horizontal movement exercise and may correspond to a chest press or a cable row.

For example, gravity coordinate is to detect a change in height (or posture value) based on the direction of gravity and may correspond to a squat or a chest press.

According to various embodiments, the electronic device 101 may identify the type of movement for each body part corresponding to the activity to be performed. Various embodiments of the type of movement for each body part according to the type of movement may be as shown in Table 2 below. Table 2 is merely an example of the type of movement for each body part according to the type of activity, but is not limited thereto.

**Table 2**

| Activity type | Movement type | | |
|---|---|---|---|
| | Head | Upper | Lower |
| Stretching | Gravity coordinates | Gravity coordinates | Gravity coordinates |
| Rope jump | Vertical movement/ Energy level | Zero-cross | Energy level |
| Squat | Gravity coordinates | Vertical movement | Gravity coordinates |
| Plank | Gravity coordinates | Gravity coordinates/ Energy level | Gravity coordinates |
| Push-up | Vertical movement | Zero-cross | Vertical movement |
| Sit-up | Zero-cross/ Vertical movement | Zero-cross | Gravity coordinates |
| Pull-up | Vertical movement | Energy level | Vertical movement |

According to various embodiments, the electronic device 101 may identify the type of the movement for each body part according to the type of the activity to be performed by the user (e.g., a trainee or a trainer) and may transmit an activation command to the wearable device (or sensor) that may detect the type of movement for each body part.

According to various embodiments, the electronic device 101 may identify the type of movement for each body part based on the sensing value obtained through at least one sensor and may identify the type of the activity performed by the user based on the identified type of movement for each body part.

FIG. 9A is a view illustrating a feedback providing operation according to an embodiment of the disclosure.

Referring to FIG. 9A, a trainee's electronic device (e.g., the electronic device 101 of FIG. 1, the processor 120 of FIG. 1, or the electronic device 101-1 or 101-3 of FIG. 2) may provide the feedback received from a server (e.g., the server 106 of FIG. 1 or the server 106 of FIG. 2). For example, the feedback may include a feedback image 910. The feedback image 910 may include a posture image 911 of the trainee and an image 912 for posture correction.

According to various embodiments, the image 912 for posture correction may be obtained in real time through a camera included in the trainer's device group and may be transmitted from the trainer's electronic device (e.g., the electronic device 101-2 of FIG. 2) to the server through a multimedia channel (e.g., the multimedia channel 310 of FIG. 3) and may be transmitted from the server to the trainee's electronic device (i.e., electronic device 101) through a multimedia channel (e.g., the multimedia channel 320 of FIG. 3). According to another embodiment, the image 912 for posture correction may be selected for posture correction, from among a plurality of feedbacks stored in the database of the server according to a result of analysis of the trainee's exercise activity based on the sensing value received by the server from the trainee's electronic device (i.e., electronic device 101) and transmitted through the multimedia channel (e.g., the multimedia channel 320 of FIG. 3) to the trainee's electronic device (i.e., electronic device 101).

According to various embodiments, the feedback may further include voice feedback which may be provided together with the feedback image 910. For example, the voice feedback may be obtained in real time via a microphone included in the trainer's device group and may be stored in the server.

FIG. 9B is a view illustrating a feedback providing operation according to an embodiment of the disclosure.

Referring to FIG. 9B, a trainee's electronic device (e.g., the electronic device 101 of FIG. 1, the processor 120 of FIG. 1, or the electronic device 101-1 or 101-3 of FIG. 2) may provide the feedback received from a server (e.g., the server 106 of FIG. 1 or the server 106 of FIG. 2). For example, the feedback may include a feedback image 920. The feedback image 920 may include an image 921 for posture correction.

According to various embodiments, the image 921 for posture correction may be generated based on a sensor value obtained in real time through at least one sensor included in the trainer's device group.

For example, if a sensing value obtained from at least one sensor of the wearable device worn by the trainer is transmitted from the trainer's electronic device (e.g., the electronic device 101-2 of FIG. 2) to the server through a health channel (e.g., the health channel 311 of FIG. 3), the server may generate an image 921 for posture correction by performing image processing based on the sensing value and may transmit the image 921 for posture correction to the trainee's electronic device (i.e., electronic device 101) through a multimedia channel (e.g., the multimedia channel 320 of FIG. 3).

In another embodiment, if the server transmits the sensing value to the trainee's electronic device (i.e., electronic device 101) through a health channel (e.g., the health channel 321 of FIG. 3), the trainee's electronic device (i.e., electronic device 101) may generate an image 921 for posture correction based on the received sensing value and provide the image 921 to the trainee.

According to another embodiment, the server may analyze the trainee's exercise activity based on the sensing value received from the trainee's electronic device (i.e., electronic device 101) and select the image 921 for posture correction according to the result of analysis from among a plurality of feedbacks stored in the database of the server and transmit the image 921 through the multimedia channel (e.g., the multimedia channel 320 of FIG. 3) to the trainee's electronic device (i.e., electronic device 101).

According to various embodiments, the feedback may further include voice feedback which may be provided together with the feedback image 920. For example, the voice feedback may be obtained in real time via a microphone included in the trainer's device group and may be stored in the server.

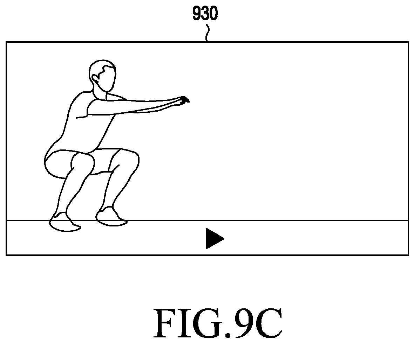

FIG. 9C is a view illustrating a feedback providing operation according to an embodiment of the disclosure.

Referring to FIG. 9C, a trainee's electronic device (e.g., the electronic device 101 of FIG. 1, the processor 120 of FIG. 1, or the electronic device 101-1 or 101-3 of FIG. 2) may provide the feedback received from a server (e.g., the server 106 of FIG. 1 or the server 106 of FIG. 2). For example, the feedback may include control information for controlling the trainee's exercise image 930, the trainer's voice feedback or text feedback.

For example, the trainer's electronic device (e.g., the electronic device 101-2 of FIG. 2) may transmit, through a multimedia channel (e.g., the multimedia channel 310 of FIG. 3) to the server, control information for playing, pausing, or controlling the playback speed of, the image 930 obtained as the trainee does exercise, as input by the trainer, the voice feedback, and/or the text feedback, and the server may transmit the feedback, received from the trainer's electronic device, through a multimedia channel (e.g., the multimedia channel 320 of FIG. 3) to the trainee's electronic device (i.e., electronic device 101), so that the trainee's electronic device (i.e., electronic device 101) may provide feedback to the trainee in real time. For example, the trainee's electronic device (i.e., electronic device 101) may play, pause, or control the playback speed of the trainee's exercise image (i.e., image 930) by the control information received from the server and may provide the trainer's voice feedback or text feedback together.

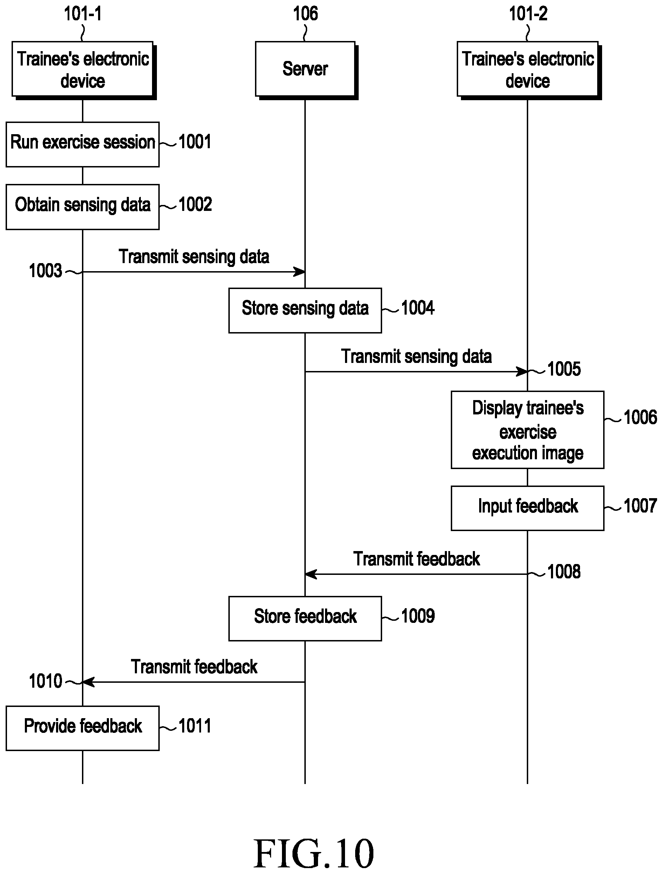

FIG. 10 is a view illustrating a real-time feedback providing operation according to an embodiment of the disclosure.

Referring to FIG. 10, a trainee's electronic device (e.g., the electronic device 101-1 or 101-3 of FIG. 2) may execute an exercise session in operation 1001. For example, the trainee's electronic device (i.e., electronic device 101-1) may execute the exercise session according to the execution of the fitness service application by the trainee, login, and an exercise start command.

According to various embodiments, if the trainee's electronic device (i.e., electronic device 101-1) accesses the server 106 through login, the server 106 (e.g., the server 106 of FIG. 1 or the server 106 of FIG. 2) may identify a plurality of exercises included in the exercise session based on the trainee's account and may transmit exercise information related to the exercise to be performed to the trainee's electronic device (i.e., electronic device 101-1). For example, the exercise information may include information related to movement for each body part of the exercise to be performed.

According to various embodiments, the exercise session to be executed is registered by the trainer. As the exercise session is executed, the body may be divided into a plurality of parts, at least one wearable device (e.g., the wearable devices 102-1 and 102-3 of FIG. 2) for detecting the type of movement for each part of the activity included in the exercise session may be activated, and exercise content for guiding the activity to be performed may be provided.

According to various embodiments, if a wearable device (e.g., the wearable device 102-1 of FIG. 2) of the trainee's device group and the server 106 are connected to each other, the server 106 may transmit an activation command to at least one of the trainee's wearable devices based on feedback.

According to various embodiments, in operation 1002, the trainee's electronic device (i.e., electronic device 101-1) may obtain sensing data (or sensing value). For example, the trainee's electronic device (i.e., electronic device 101-1) may obtain a sensing value from the activated wearable device or at least one sensor included in the trainee's electronic device (i.e., electronic device 101-1), for each body part (e.g., the head, upper body, or lower body).

According to various embodiments, the trainee's electronic device (i.e., electronic device 101-1) may further obtain the trainee's exercise image. For example, the trainee's exercise image may be obtained from a camera (e.g., the camera module 180 of FIG. 1) included in the trainee's electronic device (i.e., electronic device 101-1) or may be received from an external camera to the trainee's electronic device (i.e., electronic device 101-1).

According to various embodiments, in operation 1003, the trainee's electronic device (i.e., electronic device 101-1) may transmit sensing data to the server 106. According to various embodiments, the trainee's electronic device (i.e., electronic device 101-1) may transmit the trainee's exercise image to the server 106.

According to various embodiments, in operation 1004, the server 106 may store sensing data received from the trainee's electronic device (i.e., electronic device 101-1).

According to various embodiments, the server 106 may image-process the sensing data received from the trainee's electronic device (i.e., electronic device 101-1), generating the trainee's exercise image.

According to various embodiments, the server 106 may obtain a result of comparison between a reference sensing value stored for the performed exercise and the sensing data received from the trainee's electronic device (i.e., electronic device 101-1) and may store the obtained result of comparison in a memory. For example, the obtained result of comparison may include results for the size, accuracy, and persistence of the activity and/or results of prediction for calories burned.

According to various embodiments, in operation 1005, the server 106 may transmit the received sensing data to the trainer's electronic device (i.e., electronic device 101-2). According to various embodiments, the server 106 may transmit, to the trainee's electronic device (i.e., electronic device 101-2), the result of comparison between a reference sensing value stored for the performed exercise and the sensing data received from the trainee's electronic device (i.e., electronic device 101-1).

According to various embodiments, in operation 1006, the trainer's electronic device (i.e., electronic device 101-2) may display the trainee's exercise execution image. For example, the trainee's exercise execution image may be an image obtained from the trainee's electronic device (i.e., electronic device 101-1) or may be generated as the server 106 image-process the sensing data received from the trainee's electronic device (i.e., electronic device 101-1).

According to various embodiments, the trainee's exercise execution image may be displayed as an image including the actual trainee or may be displayed as the movement of a virtual exercise object, such as a character.

According to various embodiments, in operation 1007, the trainer's electronic device (i.e., electronic device 101-2) may receive feedback from the trainer. According to various embodiments, the trainer may evaluate the trainee's athletic ability from the difference between the sensing data received from the server 106 and the reference sensing value and may input feedback, such as correcting the exercise posture, adjusting the intensity of the exercise, or changing the type of exercise, based on the trainee's athletic ability. For example, the feedback may include at least one of image feedback, voice feedback, or text feedback.

According to various embodiments, in operation 1008, the trainer's electronic device (i.e., electronic device 101-2) may transmit the feedback to the server 106.

According to various embodiments, in operation 1009, the server 106 may store the feedback received from the trainer's electronic device (i.e., electronic device 101-2). According to various embodiments, the server 106 may store the feedback received from the trainer's electronic device (i.e., electronic device 101-2) in the trainee's account. For example, the server 106 may store feedback, such as posture correction feedback, exercise intensity adjustment, or exercise type change, in the trainee's account and may apply it in subsequent exercises.

According to various embodiments, in operation 1010, the server 106 may transmit the feedback to the trainer's electronic device (i.e., electronic device 101-2).

According to various embodiments, in operation 1011, the trainee's electronic device (i.e., electronic device 101) may provide the feedback received from the server 106. For example, the trainee's electronic device (i.e., electronic device 101-1) may output the received feedback or transmit the feedback to an external electronic device to allow the external electronic device to output the received feedback.

According to various embodiments, the server 106 may transmit/receive the sensing value to/from the trainee's electronic device (i.e., electronic device 101-1) or the trainer's electronic device (i.e., electronic device 101-2) through a health channel (e.g., the health channel 311 or 321 of FIG. 3) and may transmit/receive at least one of image data, voice data, or text data to/from the trainee's electronic device (i.e., electronic device 101-1) or the trainer's electronic device (i.e., electronic device 101-2) through a multimedia channel (e.g., the multimedia channel 310 or 320).

Although it is shown and described in connection with FIG. 10 that the feedback is input from the trainer's electronic device (i.e., electronic device 101-2), according to various embodiments, the server 106 may generate feedback based on the sensing value received from the trainee's electronic device (i.e., electronic device 101-1) and transmit the feedback to the trainee's electronic device (i.e., electronic device 101-1).

According to various embodiments, an electronic device may comprise at least one sensor, a communication module, and at least one processor operatively connected with the at least one sensor and the communication module, wherein the at least one processor may be configured to, if exercise information related to an exercise to be performed is received from a server through the communication module, identify a movement for each part of a body to be performed based on the exercise information, transmit an activation command to at least one of a plurality of connected wearable devices through the communication module based on the identified movement, transmit, to the server, at least one of a sensing value detected through the at least one sensor or a sensing value received from the at least one wearable device, and provide feedback received from the server.

According to various embodiments, the at least one processor may be configured to divide the body into a plurality of parts and identify a type of at least one movement included in each of the plurality of parts based on the exercise information.

According to various embodiments, the at least one processor may be configured to identify a reference sensing value corresponding to the type of the movement for each body part, compare at least one of the sensing value detected through the at least one sensor or the sensing value received from the at least one wearable device with the reference sensing value, and transmit a result of the comparison to the server.

According to various embodiments, the at least one processor may be configured to transmit, through the communication module, at least one of the sensing value detected through the at least one sensor or the sensing value received from the at least one wearable device to a first channel and at least one of image data, voice data, or text data to a second channel different from the first channel.

According to various embodiments, the feedback may include at least one of a voice feedback or visual feedback related to at least one of posture correction or exercise intensity.

According to various embodiments, the electronic device may further comprise a display. The at least one processor may be configured to if the exercise information is received, display an image related to the exercise to be performed on the display based on the exercise information, and if the feedback is received, display the visual feedback on the display.

According to various embodiments, the feedback may be input from an external electronic device connected with the server.

According to various embodiments, the at least one processor may be configured to transmit a control command for providing a vibration feedback to at least some of the at least one activated wearable device based on the received feedback.

According to various embodiments, a method for controlling an electronic device may comprise, if exercise information related to an exercise to be performed is received from a server, identifying a movement for each part of a body to be performed based on the exercise information, transmitting an activation command to at least one of a plurality of connected wearable devices based on the identified movement, transmitting, to the server, at least one of a sensing value detected through at least one sensor or a sensing value received from the at least one wearable device, and providing feedback received from the server.

According to various embodiments, identifying the movement for each part of the body may include dividing the body into a plurality of parts and identifying a type of at least one movement included in each of the plurality of parts based on the exercise information.

According to various embodiments, identifying the type of the movement for each body part may include identifying a reference sensing value corresponding to the movement for each body part, comparing at least one of the sensing value detected through the at least one sensor or the sensing value received from the at least one wearable device with the reference sensing value, and transmitting a result of the comparison to the server.

According to various embodiments, transmitting to the server may include transmitting, to a first channel, at least one of a sensing value detected through the at least one sensor or a sensing value received from the at least one wearable device, and transmitting, to a second channel different from the first channel, at least one of image data, voice data, or text data.

According to various embodiments, the feedback may include at least one of a voice feedback or visual feedback related to at least one of posture correction or exercise intensity.

According to various embodiments, the method may further comprise, if the exercise information is received, displaying an image related to the exercise to be performed on a display based on the exercise information, and if the feedback is received, displaying the visual feedback on the display.

According to various embodiments, the feedback may be input from an external electronic device connected with the server.

According to various embodiments, the method may further comprise transmitting a control command for providing a vibration feedback to at least some of the at least one activated wearable device based on the received feedback.

According to various embodiments, a server may comprise a memory, a communication module, and at least one processor operatively connected with the memory and the communication module. The at least one processor may be configured to identify an exercise to be performed based on a first user's account, transmit exercise information including information related to a movement for each body part of the exercise to be performed through the communication module to the first user's electronic device, receive at least one of a sensing value or the first user's exercise information from the first user's electronic device, transmit at least one of the received sensing value or the first user's exercise image to a second user's electronic device and, upon receiving feedback information from the second user's electronic device, transmit the feedback information to the first user's electronic device.

According to various embodiments, the at least one processor may be configured to store a result of comparison between the received sensing value and a reference sensing value corresponding to the movement for each body part of the exercise and the feedback information in the memory.

According to various embodiments, the at least one processor may be configured to transmit/receive the sensing value to/from the first user's electronic device or the second user's electronic device through a first channel and at least one of image data, voice data, or text data to/from the first user's electronic device or the second user's electronic device through a second channel different from the first channel.

According to various embodiments, the at least one processor may be configured to transmit an activation command to at least one wearable device of the first user's plurality of wearable devices based on the movement for each body part of the exercise to be performed.

The electronic device according to various embodiments may be one of various types of electronic devices. The electronic devices may include, for example, a portable communication device (e.g., a smart phone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program products may be traded as commodities between sellers and buyers. The computer program product may be distributed in the form of a machine-readable storage medium (e.g., compact disc read only memory (CD-ROM)), or be distributed (e.g., downloaded or uploaded) online via an application store (e.g., Play Store^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. Some of the plurality of entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

While the disclosure has been shown and described with reference to various embodiments thereof, it will be understood by those skilled in the art that various changes in form and details may be made therein without departing from the spirit and scope of the disclosure as defined by the appended claims and their equivalents.

## Claims

1. An electronic device, comprising:
at least one sensor;
a communication circuitry; and
at least one processor operatively connected with the at least one sensor and the communication circuitry, the at least one processor being configured to:
in response to exercise information related to an exercise to be performed being received from a server through the communication circuitry, identify a movement for each part of a body to be performed based on the exercise information,
transmit an activation command to at least one wearable device of a plurality of connected wearable devices through the communication circuitry based on the identified movement,
transmit, to the server, at least one of a sensing value detected through the at least one sensor or a sensing value received from the at least one wearable device,
receive, from the server, feedback; and
provide the received feedback.

2. The electronic device of claim 1, wherein the at least one processor is further configured to divide the body into a plurality of parts and identify a type of at least one movement included in each of the plurality of parts based on the exercise information.

3. The electronic device of claim 2, wherein the at least one processor is further configured to:
identify a reference sensing value corresponding to the type of the at least one movement for each body part,
compare at least one of the sensing value detected through the at least one sensor or the sensing value received from the at least one wearable device with the reference sensing value, and
transmit a result of the comparison to the server.

4. The electronic device of claim 1, wherein the at least one processor is further configured to transmit, through the communication circuitry, at least one of the sensing value detected through the at least one sensor or the sensing value received from the at least one activated wearable device to a first channel and at least one of image data, voice data, or text data to a second channel different from the first channel.

5. The electronic device of claim 1, wherein the feedback comprises at least one of a voice feedback or visual feedback related to at least one of posture correction or exercise intensity.

6. The electronic device of claim 5, further comprising a display,
wherein the at least one processor is configured to:
in response to the exercise information being received, display an image related to the exercise to be performed on the display based on the exercise information, and
in response to the feedback being received, display the visual feedback on the display.

7. The electronic device of claim 1, wherein the feedback is received from an external electronic device connected with the server.

8. The electronic device of claim 1, wherein the at least one processor is further configured to transmit a control command for providing vibration feedback to at least some of the at least one activated wearable device based on the received feedback.

9. A method for controlling an electronic device, the method comprising:
in response to exercise information related to an exercise to be performed being received from a server, identifying a movement for each part of a body to be performed based on the exercise information;
transmitting an activation command to at least one wearable device of a plurality of connected wearable devices based on the identified movement;
transmitting, to the server, at least one of a sensing value detected through at least one sensor or a sensing value received from the at least one wearable device;
receiving, from the server, feedback; and
providing the received feedback.

10. The method of claim 9, wherein the identifying of the movement for each part of the body comprises:
dividing the body into a plurality of parts; and
identifying a type of at least one movement included in each of the plurality of parts based on the exercise information.

11. The method of claim 10, wherein the identifying of the type of the at least one movement for each body part comprises:
identifying a reference sensing value corresponding to the movement for each body part;
comparing at least one of the sensing value detected through the at least one sensor or the sensing value received from the at least one wearable device with the reference sensing value; and
transmitting a result of the comparison to the server.

12. The method of claim 9, wherein the transmitting to the server comprises:
transmitting, to a first channel, at least one of a sensing value detected through the at least one sensor or a sensing value received from the at least one activated wearable device; and
transmitting, to a second channel different from the first channel, at least one of image data, voice data, or text data.

13. The method of claim 9, wherein the feedback comprises at least one of a voice feedback or visual feedback related to at least one of posture correction or exercise intensity.

14. The method of claim 13, further comprising:
in response to the exercise information being received, displaying an image related to the exercise to be performed on a display based on the exercise information; and
in response to the feedback being received, displaying the visual feedback on the display.

15. The method of claim 9, wherein the feedback is received from an external electronic device connected with the server.
